(19) Europäisches Patentamt
European Patent Office
Office européen des brevets

(11) Publication number: 0 337 937 A2

# (12) EUROPEAN PATENT APPLICATION

(21) Application number: 89810196.9

(22) Date of filing: 14.03.89

(51) Int. Cl.⁴: **C 07 C 59/00**
C 11 D 1/08, C 08 G 65/28,
C 08 G 65/32

(30) Priority: 14.03.88 US 167828

(43) Date of publication of application:
18.10.89 Bulletin 89/42

(84) Designated Contracting States:
BE CH DE FR GB IT LI NL

(71) Applicant: SANDOZ AG
Lichtstrasse 35
CH-4002 Basel (CH)

(84) Designated Contracting States:
BE CH FR GB IT LI NL

(71) Applicant: SANDOZ-PATENT-GMBH
Humboldtstrasse 3
D-7850 Lörrach (DE)

(84) Designated Contracting States: DE

(72) Inventor: Casciani, Robert
9615 Providence Forest Lane
Charlotte, NC 28226 (US)

The application is published incomplete as filed (Article 93 (2) EPC). The point in the description at which the omission obviously occurs has been left blank.

## (54) Carboxylates and ethoxylates of alkyl and alkenyl catechols.

(57) The invention relates to novel ethoxylates and novel carboxylates of alkyl and alkenyl catechols and to heir use as surface active agents. The novel ethoxylates of alkyl and alkenyl catechols are especially useful as self-sequestering surfactants in detergent compositions.

EP 0 337 937 A2

## Description

### CARBOXYLATES AND ETHOXYLATES OF ALKYL AND ALKENYL CATECHOLS

This invention relates to novel carboxylates and novel ethoxylates of alkyl and alkenyl catechols and to their use as surface active agents and to their use as self-sequestering surfactants.

Synthetic surface active agents, e.g., detergents, can be characterized as compounds which incorporate within the molecular structure thereof a hydrophobic moiety, typically a long-chain alkyl moiety, and also a hydrophilic moiety which, because of being polar in character or having ionic charge, is capable of interaction with water molecules.

As is well known in the art, the detergent properties of conventional surfactants are considerably reduced when such surfactants are employed in hard water. The reduction in detersive properties is due to the presence of multivalent ions, such as calcium and magnesium cations, in the water. To overcome this problem, it is well known to add a sequestering compound to the detergent composition, the primary function of which is to form a complex with the multivalent ions of the water and thereby compensate for the disadvantage of employing hard water.

However, a large number of sequestering compounds, e.g. sodium tripolyphosphate and nitrilotriacetic acid, have the serious drawback of causing eutrofication phenomena, and thereby adversely affect the environment.

A further disadvantage of conventional detergent compositions, i.e., those containing both a surface active agent and a sequestering compound, is that a separate synthesis is required for the two different compounds, which compounds are then mixed in various proportions, depending upon their ultimate use.

According to the invention, there is provided one or more compounds of formula I

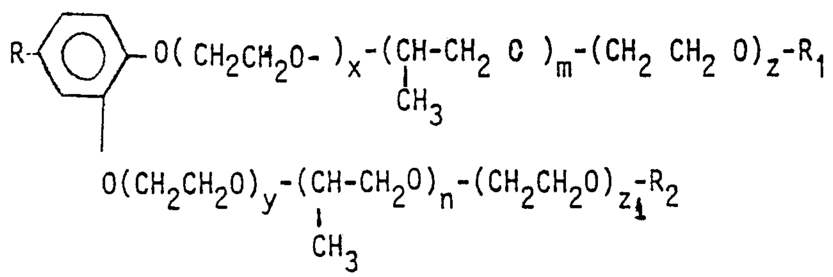

(I)

in which each of $R_1$ and $R_2$ independently is selected from hydrogen and-$(CH_2)_a$-COOX where a is an integer of 1 to 3 inclusive and X is hydrogen, an alkali metal cation or ammonium;
R is linear or branched $C_{8-22}$alkyl or linear or branched $C_{8-22}$alkenyl;
m is 0 to 20 inclusive;
n is 0 to 20 inclusive;
z is 0 to 30 inclusive;
$z_1$ is 0 to 30 inclusive; and
each of x and y is, independently, 1 to 49;
with the proviso
that the sum of x, y, z, $z_1$, m and n is 2 to 160.

Preferred compounds of formula I are those of formula Ia

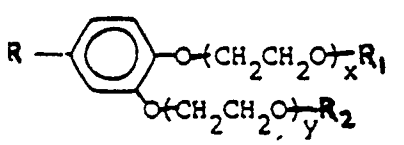

(Ia)

where R, x, y, $R_1$ and $R_2$ are as defined above, with the proviso that $x + y$ is 3 to 50.

For the avoidance of doubt where a range is given,the number defining the range is included in the said range.

R is preferably R′ where R′ is straight or branched chain $C_{8-22}$alkyl; more preferably R is R″ where R″ is $C_{8-14}$alkyl; most preferably R is R‴ where R‴ is $C_{8-12}$ alkyl.

Preferably x and y are x′ and y′ where each of x′ and y′ independently is an integer from 1 to 29 with the proviso that the sum of x and y is from 3 to 30. More preferably x and y are x″ and y″ where each of x″ and y″ independently is an integer from 1 to 19 with the proviso that the sum of x and y is from 3 to 20. Host preferably x and y are x‴ and y‴ where each of x‴ and y‴ independently is an integer from 1 to 14 with the proviso that the sum of x‴ and y‴ is 3 to 15.

Preferably $R_1$ and $R_2$ are hydrogen or -$CH_2COOX'$ where X′ is hydrogen, Na or K or ammonium.

More preferred compounds of formula I are those of formula Ib

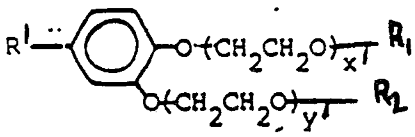

(Ib)

in which the symbols are as defined above.

Most preferred compounds of formula I are those of formula Ic

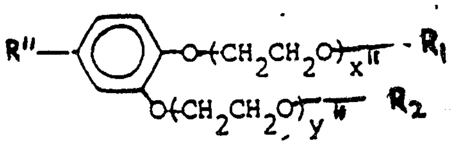

(Ic)

in which the symbols are as defined above.

Especially preferred compounds of formula I are those of formula Id

(Id)

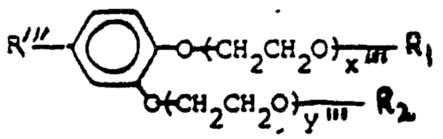

in which the symbols are as defined above.

For the avoidance of doubt, the definitions of x′ and y′; x″ and y″ and x‴ and y‴ include their provisos.

Further, according to the invention, there is provided a process for preparing a compound of formula I defined above comprising a) reacting one mole a compound of formula II

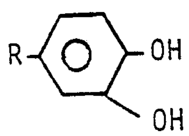

(II)

with $x + y + z^{+z_1}$ moles of ethylene oxide; and with m + n moles of propylene oxide;
optionally followed by
b) carboxylating with one or two moles of
$Hal-(CH_2)_a-COOH$
where Hal is chloro or bromo;
optionally in the presence of a strong base, in which the symbols are as defined above.

Thus, the novel alkyl and alkenyl catechol ethoxylates produced by step a) may be prepared by condensing the appropriate alkyl or alkenyl catechol compound with ethylene oxide and optionally propylene oxide by adding the alkylene oxide compounds to the alcohol. Good results are obtained by adding to the alkyl or alkenyl catechol compound to be ethoxylated, a catalytic amount, e.g., from about 0.2 % to 1 %, preferably 0.3 % to 0.75 %, by weight of the total amount of reactants, including the respective alkylene oxides, of an alkaline catalyst. Catalysts which may be employed include alkali metal hydroxides, sodium ethoxide, sodium methoxide, alkali metal acetates and dimethylamine, and mixtures thereof. Preferred catalysts are the alkali metal hydroxides, more preferably sodium hydroxide and potassium hydroxide. Other types of catalysts commonly used for alkylene oxide condensation reactions may also be employed.

Optionally, a small amount of a reducing agent may be added to the alkyl or alkenyl catechol compound to be ethoxylated to minimise coloration of the resulting ethoxylated alkyl or alkenyl catechol compound. Suitable reducing agents which may be employed include sodium borohydride, lithium aluminium hydride, diborane and the like, preferably sodium borohydride.

An amount of ethylene oxide (and optionally propylene oxide) calculated to provide the desired degree of ethoxylation (and propoxylation if present) is then introduced in the resulting mixture and is allowed to react until all of the ethylene oxide (and optionally propylene oxide) is consumed, as indicated by a drop in reaction pressure. Customarily, the ethoxylated product is finally treated with weak acid, e.g., glacial acetic acid, to neutralise any basic catalyst residues.

The variation in the number of ethylene oxide and optional propylene oxide moieties is not critical as long as the average for the number of units in each block is within the limits set out for the m, n, z, $z_1$, x and y terms in formula I above, which terms, as average values, are other than whole numbers in some instances.

The ethoxylation (and optional propoxylation) procedure is conducted at an elevated temperature and pressure. Suitable reaction temperatures are from about 120°C to about 220°C, preferably 130°C to 180°C, and more preferably 140°C to 160°C. A suitable reaction pressure is achieved by introducing to the reaction vessel the required amount of ethylene oxide (and optionally propylene oxide) which has a high vapour pressure at the desired reaction temperature. The pressure serves as a measure of the degree of reaction and the ethoxylation ( and propoxylation) is considered to be complete when the pressure no longer decreases

with time.

For best results, it is desirable to carry out the ethoxylation (and any propoxylation) under relatively moisture-free conditions and to avoid side reactions which form water. To dry the reaction vessel and connection, they may be swept out with a dry, oxygen-free gas, e.g., nitrogen, before introducing the charge. The catalyst or catalyst mixture should also be dry, or substantially so. The ethylene oxide (and propylene oxide) should preferably be purified to remove moisture and any impurities which are capable of entering into side reactions which yield water.

To prepare the corresponding carboxyalkylated derivatives, the resulting alkyl and alkenyl catechol ethoxylates are then carboxyalkylated by the Williamson synthesis, involving reaction with an appropriate chloro- or bromocarboxylic acid or a salt thereof in the presence of a strong base, e.g., sodium hydroxide, sodium carbonate, etc., metallic sodium, or by catalytic oxidation. The carboxyalkylation is conducted at a temperature of between 20° and 100° C, preferably between 50° and 90° C, and at a mole ratio of between 0.3:1 and 2.5;1, preferably between 1:1 and 2:1, of chloro-or bromocarboxylic acid or a salt thereof, e.g., sodium monochloroacetate, to alkyl or alkenyl catechol ethoxylate. Such reactions are ordinarily not complete; hence, the reaction products often contain minor amounts of uncarboxylated alkyl or alkenyl catechol ethoxylate. While methods are available for separating the uncarboxylated material as well as for assuring the essentially complete carboxyalkylation, they are usually tedious and expensive. Fortunately, it has been found that minor proportions of such uncarboxyalkylated material are not particularly harmful, and may even be advantageous. The carboxyalkylation involving reaction with an appropriate chloro- or bromocarboxylic acid or a salt thereof in the presence of a strong base is preferred for completeness. Preferably the carboxyalkyl group is a carboxymethyl group.

Alternatively, the corresponding carboxyalkylated derivatives of formula I wherein $R_2$ is $-CH_2CH_2-$ may be prepared via base catalysed 1,4- addition to an appropriate $\alpha,\beta$-unsaturated compound such as methyl acrylate or acrylamide followed by hydrolysis. Moreover, the corresponding carboxyalkylated derivatives of formula I wherein $R_2$ is $-CH_2CH_2CH_2-$ may be prepared by a reaction involving alkoxylate addition to an appropriate lactone which yields the corresponding carboxylate.

The compounds of formula I are surface active agents. The uses to which surface active agents can be put are numerous and well known and, as a result, the possible applications of these new compounds are extremely varied. Thus, the surface active agents of the present invention are suitable as emulsifiers, dispersing agents, wetting agents, levelling agents and the like in the textile, leather, paper, lacquer, personal care, e.g., toiletries, cosmetics, etc., and rubber Industries. For example, they can be used as wetting agents in spinning, twisting, weaving and dyeing operations in the textile industry, especially in the presence of hard water. In addition, they can be utilised for converting liquid or solid substances which, per se, are insoluble in water (such as hydrocarbons, higher alcohols, oils, fats, waxes and resins) into creamy emulsions, clear solutions or fine, stable dispersions.

2,4-D and the like as well as for use as additives to petroleum products, hydraulic fluids, lubricating oils, cutting oils and greases. They may also be employed as coating aids for use in coating compositions comprising a hydrophilic, film-forming colloid; as tackifiers in the adhesive layer of adhesive tapes in, e.g., the photographic industry; and as foaming agents and dispersing agents in a wide variety of mining applications (i.e., ore flotation).

Furthermore, because of their ability to complex metal ions, the compounds of formula I can be used to remove heavy metal contaminants from waste water. These materials are especially useful in this context due to their surface active properties. Therefore, processes such as solvent extraction can be facilitated by utilising the compounds of the instant invention.

The compounds of formula I are useful as self-sequestering surfactants in detergent compositions. Thus, in view of their detersive properties, they may be employed as the sole surfactant component in said detergent compositions and, as a result of their sequestering capabilities, they may be employed as the sole sequestering component, i.e., the detergent compositions need not contain a different and separate sequestering compound. As self-sequestering surfactants, the alkyl and alkenyl catechol ethoxylates of the instant invention form soluble complexes with calcium and magnesium cations sufficiently stable to eliminate the negative influences on the detersive properties of the compositions but not so stable as to cause ecological imbalance because of an accumulation of ions. Such detergent compositions will normally contain from 5 % to 50 % of the compound of formula I preferably from 10 % to 50 %, and more preferably from 15 % to 50 %.

The compounds of formula I can advantageously be employed in detergent compositions which already contain a conventional surfactant, e.g., an anionic, non-ionic, ampholytic or zwitterionic surfactant, or mixtures thereof, coupled with the additional advantage that, as compared with other known sequestering compounds under equal conditions, such detergent compositions exhibit greater detersive capability without concomitant harmful environmental effects or any other drawbacks and disadvantages of the known sequestering compounds. Preferred anionic surfactants are:

1) alkylbenzenesulphonates, such as sodium and potassium salts having a branched or straight chain alkyl portion of about 9 to about 15 carbon atoms;

2) alkyl sulphates, such as the sodium and triethanolammonium salts of $C_{10-20}$ alkyl sulphuric acids, prepared by sulphating the alcohols derived from coconut oil or tallow, or prepared synthetically;

3) the alkali metal and ammonium salts of the sulphated ethoxylates of a long-chain alcohol and 3 to 5 molar proportions of ethylene oxide, e.g., the ammonium salts of an ethoxylate containing an average of 3.1 molar proportions of ethylene oxide and 1 mole of an alcohol mixture known commercially as ALFOL 1412, composed of about 2/3 n-tetradecanol and about 1/3 n-dodecanol;

4) the compounds known as "Medialans" which are amido carboxylic acids formed by condensing fatty acids of $C_8$-$C_{22}$ chain length with sarcosine, $CH_3NHCH_2COOH$;

5) alkanesulphonates, such as ammonium dodecanesulphonate;

6) alkoxyhydroxypropanesulphonates, such as the water-soluble salts of 3-dodecyloxy-2-hydroxy-1-propane-sulphonate;

7) soaps, the surface-active substances formed usually by the reaction of caustic alkalis with natural glyceridic fats and oils, generally prepared in high purity, and having the generic molecular formula RCOONa, wherein R is a straight-chain hydrocarbon group having from about 7 to about 21 carbon atoms; and

8) olefinic sulphonates, such as dodecene sulphonate, and the compounds described in USP 3,332,880. As representative of non-ionic surfactants may be mentioned:

1) the Pluronics, formed by condensing propylene oxide with propylene glycol to a molecular weight of about 600 to 2500 to form a base followed by condensing ethylene oxide to this base to the extent of about 10 to about 90 percent, total molecule basis. USP 2,674,619 and USP 2,677,700 describe operable compounds;

2) compounds formed by the simultaneous polymerization of propylene oxide and ethylene oxide and containing randomly positioned oxypropylene and oxyethylene groups. These and related compounds are described in USP 2,979,528, USP 3,036,118, USP 3,022,335, USP 3,036,130 and USP 3,048,548;

3) alkyl phenols having 9 to 12 carbon atoms in the alkyl portion (straight or branched) ethoxylated with 4-10 molar proportions of ethylene oxide; and

4) ethoxylates of fatty alcohols having 8 to 18 carbon atoms per molecule and 5-30 molar proportions of oxyethylene groups.

As an example of an ampholytic surfactant may be mentioned the hydroxyalkyl methyl taurates, while cocodimethyl sulphopropyl betaine is exemplary of a zwitterionic surfactant.

Preferably in detergent compositions which contain a compound of formula I and a second surfactant, the amount of compound of formula I will be 60-95 % and the amount of second surfactant will be 5-40 % by weight of the mixture; more preferably the amount of the compound of formula I will be 70-90 % and the amount of the second surfactant will be between 10 and 30 %.

When a compound of formula I is employed in detergent compositions which contain a second different sequestering compound having one or more of the above discussed drawbacks and disadvantages, the concentration of the different sequestering compound can be reduced, thereby reducing the toxic and polluting properties of said different sequestering compound. Representative compound can be reduced, thereby reducing the toxic and polluting properties of said different sequestering compound. Representative inorganic sequestering compounds are, e.g., water soluble salts of pyrophosphates, orthophosphates, polyphosphates, phosphonates, carbonates, bicarbonates and silicates. As representative of organic sequestering compounds may be mentioned the alkali metal, ammonium and substituted ammonium polyacetates, carboxylates, polycarboxylates and polyhydroxy-sulfonates.

In detergent compositions which contain a compound of formula I and a second sequestering compound, preferably the amount of the compound of formula I is from 40-95 % and of the second sequestering agent of a composition is from 5 to 60 %.

The detergent compositions can optionally contain all manner of additional materials commonly found in laundering and cleaning compositions. For example, oxidizing bleaches such as sodium perborate, sodium percarbonate, etc., optionally with bleach precursors such as phthalic anhydride, may be incorporated at levels of 1 % to 25 % of the compositions. When these materials are present, the compounds of formula I can also serve as peroxide stabilizers. Their ability to sequester certain metal ions will serve to prevent the undesired decomposition of these oxidizing agents.

Defoamers such as long-chain fatty acids, silicone fluids and microcrystalline waxes may be employed alone or as mixtures at levels of 0.005 % to 5 %, preferably 0.01 % to 3 %, and most preferably 0.1 % to 1 %, of the composition.

Viscosity modifiers and anticaking agents such as the sodium salts of lower alkyl aromatic sulphonic acids and the alkali metal salts of sulphosuccinic acid and benzene sulphonic acid are conveniently employed at levels of 0.5 % to 5 %, particularly if other anionic surfactants are employed as part of the surfactant mixture.

Soil suspending agents such as sodium carboxymethyl cellulose and hydroxyethyl cellulose may also be used in amounts of 0.25 % to 5 % by weight of the composition.

Enzymes such as the proteolytic enzymes may be incorporated at levels of up to 1 % by weight, preferably from 0.25 % to 0.75 % by weight. Such enzymatic materials may be coated or pilled to aid their stability and to

minimize the formation of dust during processing and subsequent storage.
The invention will be illustrated by the following Examples:

## EXAMPLE 1

**Preparation of Dodecaethoxy nonylcatechol, a compound of formula 1a**

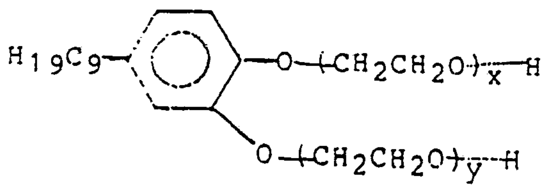

where x + y = 12.

**a) Preparation of nonylcatechol**

Into a 500 ml, 3-neck reaction vessel was placed 64.8 g (0.3125 moles) of catechol, after which time it was heated to 104°C under a nitrogen flow. After the catechol was completely melted, 3.25 g (5 % based on the weight of the catechol) of borontrifluoride-etherate was added dropwise, with stirring. After adding all of the catalyst, 63.2 g (0.25 moles) of 1-nonene was added, dropwise to the reaction mixture, the rate of addition being adjusted so as to maintain a temperature of 130°C. After all of the 1-nonene was added, the resultant mixture was allowed to react for 3 hours, after which time 100 ml of a 10 % sodium chloride solution was added to the reaction mixture. The resultant mixture was then stirred for 15 minutes at 95° to 100°C, after which time the bottom layer was separated. The mixture was again stirred at 95° to 100°C for 15 minutes, after which time the bottom layer was again separated. The organic layer was then isolated to obtain a viscous, brown oil of the formula 1b

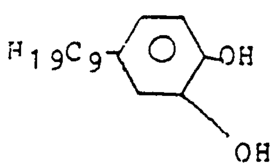

(1b)

**b) Preparation of dodecaethoxy nonylcatechol**

Into a reaction vessel containing 62 g of the compound prepared in a) above was added, with stirring, 0.1 g of sodium hydroxide catalyst. The system was then purged with nitrogen, evacuated and the temperature raised to 120°C, after which time the system was again purged and evacuated. The purging and evacuation procedure is then repeated twice more at 120°C, after which time the temperature of the reaction mixture was raised to 155°C. 140 g of ethylene oxide was then added to the reaction mixture at a rate such that a constant pressure was maintained. When the addition of ethylene oxide is complete, the reaction mixture is allowed to post-react until the pressure drops to a point where it remains constant for at least 30 minutes. The system is then cooled to 60°C and the vacuum broken with nitrogen. The remaining sodium hydroxide catalyst is then neutralized with acetic acid, after which time the reaction mixture is cooled and filtered to yield the title compound.

## EXAMPLE 2

Following essentially the last step of the procedure in preparing the compound of Example 1 and utilizing the catechol compound prepared in 1a) and the appropriate amounts of ethylene oxide, the following compounds are obtained:

**a) the compound of formula 2a**

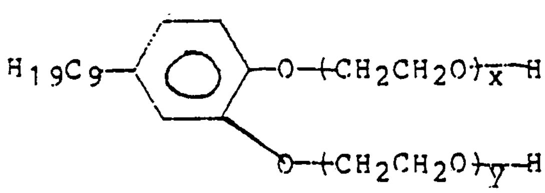

(2a)

where x + y = 3;

**b) the compound of formula 2b**

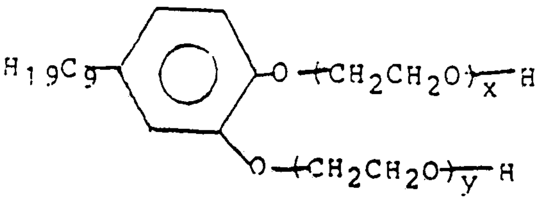

(2b)

where x + y = 6; and

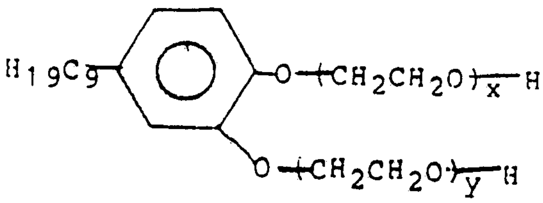

(2c)

where x + y = 9.

It should be understood that in all of the examples above, the indicated number of ethyleneoxy units are average values.

**EXAMPLE 3**

To demonstrate the ability of the catechol ethoxylates of the instant invention to sequester metal ions, they were evaluated by determining the stability constants of the complexes formed by the calcium utilizing potentiometric titrations with electrodes selective towards the calcium ion in question. During these titrations, the quantity of calcium ion complexed by a known quantity of sequestering agent was measured and the result of the measurement was used to calculate the constant of the complex (Kca) employing the following equation:

$$Kca = \frac{[Ca\ tot] - [Ca\ free]}{[Ca\ free] \times [L\ free]}$$

where L = ligand or sequestrant
and [L free] = [L tot] - [Ca tot] + [Ca free].

At high ratios of [Ca tot] / [L tot], the ligand became saturated with Ca ion and a linear increase in [Ca free] resulted. This line was extrapolated back to [Ca free] = 0 and [Ca tot] at that point represented a measure of calcium binding capacity. For purposes of comparison, the calcium binding capacity of hexaethoxy nonylphenyl was evaluated and the results appear herein below:

| | Kca | Log Kca |
|---|---|---|
| compound of Example 2b) | 32 | 1.5 |
| hexaethoxy nonylphenyl | 0 | - |

From the above results, it is clear that the catechol ethoxylate compound of the instant invention exhibits excellent calcium binding ability whereas the hexaethoxy nonylphenyl compound has no calcium binding ability.

**EXAMPLE 4**

The following represent typical formulations useful as detergent compositions:

**Solid**

| | A | B | C |
|---|---|---|---|
| Example 2b) compound | 50 | 30 | 35 |
| Sodium tripoly-phosphate | - | 20 | - |
| Sodium silicate ($Na_2O:SiO_2 = 1:2.5$) | 6 | 6 | 6 |
| LAS (linear alkylbenzene sulfonate) | - | - | 15 |
| Sodium carboxymethyl cellulose | 0.3 | 0.3 | 0.3 |
| Sodium sulfate | 24 | 24 | 24 |
| Fluorescent dye | 0.16 | 0.16 | 0.16 |
| Water | 8 | 8 | 8 |
| miscellaneous | balance | balance | balance |

**Liquid**

| | A | B | C |
|---|---|---|---|
| Example 2b) compound | - | 30 | 18 |
| Tetrapotassium pyrophosphate | - | 12 | - |
| Sodium silicate ($Na_2O:SiO_2 = 1:1.6$) | 3.8 | 3.8 | 3.8 |
| LAS | - | - | 9 |
| Sodium carboxymethyl cellulose | 0.3 | 0.3 | 0.3 |
| Perfume | 0.2 | 0.2 | 0.2 |
| Water | 65.7 | 65.7 | 65.7 |

**EXAMPLE 5**

73.7g of the compound of formula 2a of Example 2 was placed into a 3-neck reaction vessel equipped with a condensor, mechanical stirrer and thermometer. To the flask was added, sequentially with stirring, 10.56 g of potassium hydroxide and 6.4 g of sodium hydroxide. The resultant mixture was then stirred for one hour, while the temperature was maintained between 50° and 55° C, 37.4 g of sodium monochloroacetate was then added to the mixture in four equal portions over a two hour period, while the exotherm was controled to 65+/-5° C. After the addition was complete, the reaction was allowed to proceed for 20 hours, after which time the reaction mxture was diluted with 50 ml of water and 75ml of a 10 % sulphuric acid solution. Phase separation yielding an amber oil having the formula

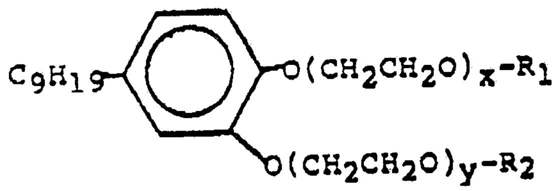

where x + y = 3;
and $R_1$ + $R_2$ = $-CH_2COOH$ (47 0/0); -H (53 0/0).

**EXAMPLE 6**

75.0g of the compound of formula 2b of Example 2 was placed into a 3-neck reaction vessel equipped with a condensor, mechanical stirrer and thermometer. To the flask was added, sequentially with stirring, 7,9 g of potassium hydroxide and 4.81 g of sodium hydroxide. The resultant mixture was then stirred for one hour, while the temperature was maintained between 50° and 55°C. 28.1 g of sodium monochloro acetate was then added to the mixture in four equal portions over a two hour period, while the exotherm was controlled to 65 +/- 5°C. After this addition was complete, the reaction was allowed to proceed for 20 hours, after which time the reaction mixture was diluted with 50 ml of water and 75 ml of a 10 0/0 sulphuric acid solution. Phase separation yielded an amber oil having the formula

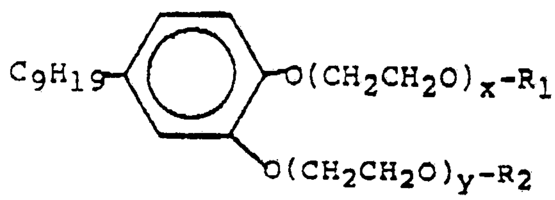

where x + y = 6;
and $R_1$ + $R_2$ $-CH_2COOH$ (52 0/0); -H (48 0/0).

**EXAMPLE 7**

63.2g of the compound of formula 2c of of Example 2 was placed into a 3-neck reaction vessel equipped with a condensor, mechanical stirrer and thermometer. To the flask was added, sequentially with stirring, 5.3 g of potassium hydroxide and 3.21 g of sodium hydroxide. The resultant mixture was then stirred for one hour, while the temperature was maintained between 50° and 55°C. 18.7 g of sodium monochloroacetate was then added to the mixture in four equal portions over a two hour period, while the exotherm was controlled to 65+/-5°C. After the addition was complete, the reaction was allowed to proceed for 20 hours, after which time the reaction mixture was diluted with 50 ml of water and 75 ml of a 10 0/0 sulphuric acid solution. Phase separation yielded an amber oil having the formula

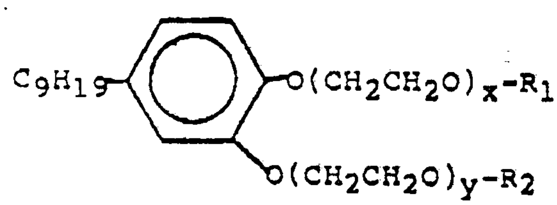

where x + y = 9;
and $R_1$ + $R_2$ = $-CH_2COOH$ (58 0/0); -H(42 0/0).

**EXAMPLE 8**

76.5g of the compound of formula 1a of Example 1 was placed into a 3-neck reaction vessel equipped with a condensor, mechanical stirrer and thermometer. To the flask was added, sequentially with stirring, 5.3 g of potassium hydroxide and 3.21 g of sodium hydroxide. The resultant mixture was then stirred for one hour, while the temperature was maintained between 50° and 55°C. 18.7 g of sodium monochloroacetate was then added to the mixture in four equal portions over a two hour period, while the exotherm was controlled to 65+/-5°C. After the addition was complete, the reaction was allowed to proceed for 20 hours, after which time the reaction mixture was diluted with 50 ml of water and 75 ml of a 10 0/0 sulphuric acid solution. Phase separation yielded an amber oil having the formula

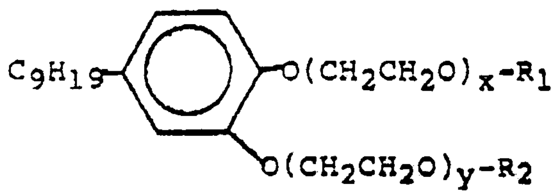

where x + y = 12;
and $R_1 + R_2$ = $-CH_2COOH$ (66 %); -H (34 %).

As to the chelating results, the following was obtained on the carboxylate set forth in Example 6 above.:

| [Ca] total | $K_{ca}$ | log $K_{ca}$ |
|---|---|---|
| 0.00099 | 134.6 | 2.13 |
| 0.00476 | 41.3 | 1.62 |
| 0.00909 | 21.9 | 1.34 |

The calcium binding exhibited by the carboxylate was particularly noteworthy at low Ca + concentrations.

## Claims

1. A compound of formula I

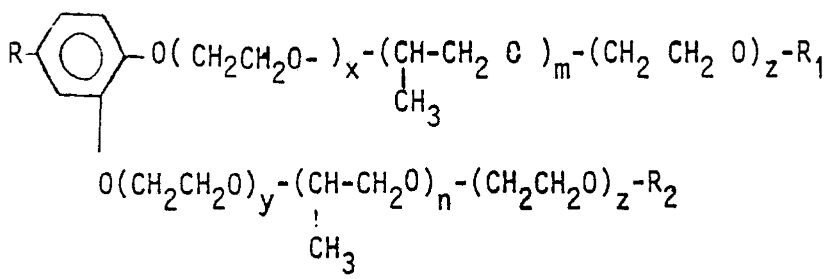

in which each of $R_1$ and $R_2$ independently is selected from hydrogen and-$(CH_2)_a$-COOX where a is an integer of 1 to 3 inclusive and X is hydrogen, an alkali metal cation or ammonium;
R is linear or branched $C_{8-22}$alkyl or linear or branched $C_{8-22}$alkenyl;
m is 0 to 30 inclusive;
n is 0 to 30 inclusive;
z is 0 to 30 inclusive;
$z_1$ is 0 to 30 inclusive;
each of x and y is, independently, 1 to 49;
with the proviso
i) that the sum of x, y, z, $z_1$, m and n is 2 to 160.

2. A compound according to Claim 1 of formula Ia

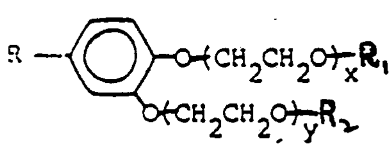 (Ia)

where R, x, y $R_1$ and $R_2$ are as defined in Claim 1.

3. A compound according to Claim 1 or Claim 2, in which R is R′ where R′ is a straight or branched claim $C_{8-22}$alkyl.

4. A compound according to any one of the preceding claims, in which x and y are x′ or y′ where each of x′ and y′ independently is an integer of 1 to 29 with the proviso that the sum of x and y is 3 to 30.

5. A compound according to any one of the preceding claims, in which $R_1$ and $R_2$ are hydrogen.

6. A compound according to Claim 1 of formula Ib

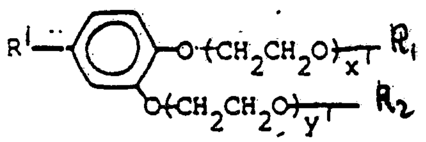

(Ib)

in which R1 and R2 are as defined in Claim 1; R′ is as defined in Claim 3; and x′ and y′ are as defined in Claim 4.

7. A compound according to Claim 1 of formula Id

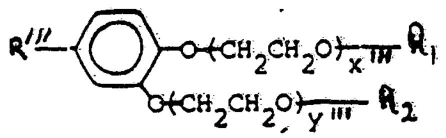

in which R1 and R2 are defined in Claim 1; R‴ is straight or branched chain $C_{8-12}$alkyl; and each of x‴ and y‴ independently, is an integer of 1 to 14; with the proviso that the sum of x‴ and y‴ is 3 to 18.

8. A compound according to Claim 1 of the formula

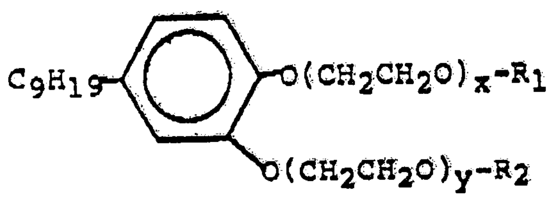

where $x + y = 3, 6, 9$, or 12, and $R_1 + R_2 = -CH_2COOH$ or - H.

9. A detergent composition comprising, as a self-sequestering surfactant component thereof, from about 5 % to about 50 % of a compound according to any one of the preceding claims, 5 % to about 50 % of a compound according to any one of the preceding claims.

10. A detergent composition according to any one of Claims 13 to 15 comprising, in addition to a self-sequestering surfactant component thereof, an anionic, non-ionic, ampholytic or zwitterionic surfactant, or a mixture thereof, said additional surfactant being present in an amount of from about 5 % to about 40 % by weight of the mixture of the self-sequestering surfactant and additional surfactant.